# EUROPEAN PATENT APPLICATION

(11) **EP 4 343 708 A1**
(43) Date of publication of application: **27.03.2024**
(21) Application number: 21949563.7
(22) Date of filing: 12.07.2021
(51) Int. Cl.: G06V 10/00

(54) **METHOD AND APPARATUS FOR TRAINING MACHINE LEARNING MODELS, COMPUTER DEVICE, AND STORAGE MEDIUM**

(71) Applicant: Shanghai United Imaging Healthcare Co., Ltd., Shanghai 201807 (CN)
(72) Inventor: NI, Cheng, Shanghai 201807 (CN); LIU, Runxin, Shanghai 201807 (CN); ZHANG, Wei, Shanghai 201807 (CN); ZHANG, Kang, Shanghai 201807 (CN)
(74) Representative: Murgitroyd & Company
(86) International application number: PCT/CN2021/105777
(87) International publication number: WO 2023/283765

(57) **Abstract**

The present application relates to a method and an apparatus for training machine learning models, a computer device, and a storage medium. The method comprises: acquiring a first training sample set and a second training sample set; the training samples in the first training sample set and the second training sample set comprise medical images obtained by a medical scanning device scanning a scanned subject; on the basis of the first sample set, performing multi-round model training to obtain a first machine learning model; and on the basis of the second sample set, performing multi-round model training to obtain a second machine learning model; the first machine learning model and the second machine learning model are at least partially identical in structure, and at least some of the model parameters of the second machine learning model are used when the first machine learning model is trained and at least some of the model parameters of the first machine learning model are used when the second machine learning model is trained. By use of the present method, model accuracy can be increased without the need to share medical images.

## Description

### TECHNICAL FIELD

The present disclosure relates to the technical field of model training, and in particular, to a method and apparatus for training machine learning models, a computer device, and a storage medium.

### BACKGROUND

With the development of medical imaging devices, machine learning models for image processing of medical images have been widely used.

Generally, the training of machine learning models requires a large number of training samples. However, since the medical images involve patient privacy and data security, and the medical images cannot be shared between hospitals, there are fewer training samples for the machine learning model, resulting in poor accuracy of the machine learning model.

### SUMMARY

Based on this, to address the above technical problems, it is necessary to provide a method and apparatus for training machine learning models, a computer device, and a storage medium that can improve the accuracy of the model without sharing medical images.

A method for training machine learning models is provided. The method includes:
acquiring a first training sample set and a second training sample set, training samples in the first training sample set and the second training sample set including medical images obtained by scanning a scan subject with a medical scanning device;
performing, based on the first training sample set, multiple rounds of model training, to obtain a first machine learning model; and
performing, based on the second training sample set, multiple rounds of model training, to obtain a second machine learning model,

At least a part of the first machine learning model has a same structure as at least a part of the second machine learning model, at least a part of model parameters of the second machine learning model is used when the first machine learning model is trained, and at least a part of model parameters of the first machine learning model is used when the second machine learning model is trained.

In an embodiment, the performing, based on the first training sample set, multiple rounds of model training, to obtain the first machine learning model includes:
performing, based on first initial model parameters and the first training sample set, a first round of model training, to obtain a first initial model, at least a part of model parameters of the first initial model being used to train the second machine learning model;
performing, based on the first training sample set and at least a part of the model parameters of the present second machine learning model, an Nth round of model training, to obtain the first machine learning model, N being a positive integer greater than 1; and
terminating, if it is determined that a model index of the first machine learning model meets a first preset index, the training of the first machine learning model.

In an embodiment, a part of the first machine learning model has the same structure as a part of the second machine learning model, and the performing, based on the first training sample set and at least a part of the model parameters of the present second machine learning model, the N^{th} round of model training, to obtain the first machine learning model includes:
performing, based on the first training sample set, model parameters of the part of the present second machine learning model having the same structure, and a part of the model parameters of the first machine learning model obtained from a previous round of model training, the N^{th} round of model training, to obtain the first machine learning model.

In an embodiment, the whole of the first machine learning model has the same structure as the whole of the second machine learning model, and the performing, based on the first training sample set and at least a part of the model parameters of the present second machine learning model, the N^{th} round of model training, to obtain the first machine learning model includes:
performing, based on the first training sample set and all of the model parameters of the present second machine learning model, the N^{th} round of model training, to obtain the first machine learning model.

In an embodiment, the method further includes:
performing, if it is determined that the model index of the first machine learning model does not meet the first preset index, an (N+1)^{th} round of model training of the first machine learning model.

In an embodiment, the performing, based on the second training sample set, multiple rounds of model training, to obtain the second machine learning model includes:
performing, based on the second training sample set, and at least a part of the model parameters of the present first machine learning model, an M^{th} round of model training, to obtain the second machine learning model, M being a positive integer greater than 0; and
terminating, if it is determined that a model index of the second machine learning model meets a second preset index, the training of the second machine learning model.

In an embodiment, a part of the first machine learning model has the same structure as a part of the second machine learning model, and the performing, based on the second training sample set and at least a part of the model parameters of the present first machine learning model, the M^{th} round of model training, to obtain the second machine learning model includes:
performing, based on the second training sample set, model parameters of the part of the first initial model having the same structure, and second initial model parameters, a first round of model training, to obtain a second initial model, at least a part of model parameters of the second initial model being used to train the first machine learning model; and
continuing to perform, based on the second training sample set, model parameters of the part of the present first machine learning model having the same structure, and a part of the model parameters of the second machine learning model obtained from a previous round of model training, the model training, to obtain the second machine learning model.

In an embodiment, the whole of the first machine learning model has the same structure as the whole of the second machine learning model, and the performing, based on the second training sample set and at least a part of the model parameters of the present first machine learning model, the M^{th} round of model training, to obtain the second machine learning model includes:
performing, based on the second training sample set and all of the model parameters of the present first machine learning model, the M^{th} round of model training, to obtain the second machine learning model.

In an embodiment, the method further includes:
performing, if it is determined that the model index of the second machine learning model does not meet the second preset index, an (M+1)^{th} round of model training of the second machine learning model.

In an embodiment, the model index includes an accuracy of an output result, the first preset index includes a first preset accuracy, and the second preset index includes a second preset accuracy.

In an embodiment, the method further includes:
determining, during each round of model training, a descent gradient using a batch gradient algorithm if it is determined, according to a preset loss function, that an output result of the machine learning model does not meet a preset convergence condition, and continuing to perform the model training, and stopping, until it is determined that the output result of the machine learning model meets the preset convergence condition, the present round of model training.

In an embodiment, the acquiring the first training sample set and the second training sample set includes:
acquiring medical images from a first hospital, and generating, based on the medical images from the first hospital, the first training sample set; and
acquiring medical images from a second hospital, and generating, based on the medical images from the second hospital, the second training sample set;

The first hospital is different from the second hospital.

In an embodiment, the first machine learning model and the second machine learning model include at least one of a dose prediction model, an automatic sketching model, an efficacy evaluation model, a survival index evaluation model, a cancer screening model or a deformation registration model.

In an embodiment, the model parameters of the first machine learning model and the model parameters of the second machine learning model are transferred through a network.

Alternatively, the model parameters of the first machine learning model and the model parameters of the second machine learning model are transferred through a storage medium.

In an embodiment, the training of the first machine learning model and the training of the second machine learning model are performed on two independent networks, respectively.

In an embodiment, the training of the first machine learning model and the training of the second machine learning model are performed alternately.

In an embodiment, the first machine learning model and the second machine learning model are identical in structure and application.

In an embodiment, the first machine learning model and the second machine learning model are different in application.

In an embodiment, only model parameters are transferred during the training of the first machine learning model and the second machine learning model.

In an embodiment, the method further includes:
combining the first machine learning model and the second machine learning model, to obtain a target machine learning model.

A method for training machine learning models is provided, the method includes:
acquiring at least two training sample sets, training samples in the training sample set including medical images obtained by scanning a scan subject with a medical scanning device; and
performing, based on each of the at least two training sample sets, multiple rounds of model training, to obtain a machine learning model corresponding to a respective training sample set.

At least a part of each of at least two machine learning models has a same structure as at least a part of each of other machine learning models, and when one of the at least two machine learning models is trained, at least a part of model parameters of the part of another of the at least two machine learning models having the same structure is used.

In an embodiment, the performing, based on each of the at least two training sample sets, multiple rounds of model training, to obtain a machine learning model corresponding to a respective training sample set includes:
obtaining model parameters for a present round for each of the machine learning models, and performing, based on the training sample set corresponding to the machine learning model and the model parameters for the present round, the model training, to obtain the machine learning model, wherein the model parameters for the present round include initial model parameters, or the model parameters of the part of another machine learning model having the same structure.

In an embodiment, the training of each of the machine learning models is performed on an independent network.

In an embodiment, at least two of the machine learning models have the same configuration and the same application.

In an embodiment, each of the machine learning models has a different application.

An apparatus for training machine learning models is provided. The apparatus includes:
a sample set acquisition module configured to acquire a first training sample set and a second training sample set, training samples in the first training sample set and the second training sample set including medical images obtained by scanning a scan subject with a medical scanning device;
a first training module configured to perform, based on the first training sample set, multiple rounds of model training, to obtain a first machine learning model; and
a second training module configured to perform, based on the second training sample set, multiple rounds of model training, to obtain a second machine learning model.

At least a part of the first machine learning model has a same structure as at least a part of the second machine learning model, at least a part of model parameters of the second machine learning model is used when the first machine learning model is trained, and at least a part of model parameters of the first machine learning model is used when the second machine learning model is trained.

In an embodiment, the above first training module is specifically configured to: perform, based on first initial model parameters and the first training sample set, a first round of model training, to obtain a first initial model, at least a part of model parameters of the first initial model being used to train the second machine learning model; perform, based on the first training sample set and at least a part of the model parameters of the present second machine learning model, an N^{th} round of model training, to obtain the first machine learning model, N being a positive integer greater than 1; and terminate, if it is determined that a model index of the first machine learning model meets a first preset index, the training of the first machine learning model.

In an embodiment, a part of the first machine learning model has the same structure as a part of the second machine learning model. The above first training module is specifically configured to perform, based on the first training sample set, model parameters of the part of the present second machine learning model having the same structure, and a part of the model parameters of the first machine learning model obtained from a previous round of model training, the N^{th} round of model training, to obtain the first machine learning model.

In an embodiment, the whole of the first machine learning model has the same structure as the whole of the second machine learning model. The above first training module is specifically configured to perform, based on the first training sample set and all of the model parameters of the present second machine learning model, the N^{th} round of model training, to obtain the first machine learning model.

In an embodiment, the above first training module is further configured to perform, if it is determined that the model index of the first machine learning model does not meet the first preset index, an (N+1)^{th} round of model training of the first machine learning model.

In an embodiment, the above second training module is specifically configured to perform, based on the second training sample set, and at least a part of the model parameters of the present first machine learning model, an M^{th} round of model training, to obtain the second machine learning model, M being a positive integer greater than 0; and terminate, if it is determined that a model index of the second machine learning model meets a second preset index, the training of the second machine learning model.

In an embodiment, a part of the first machine learning model has the same structure as a part of the second machine learning model. The above second training module is specifically configured to perform, based on the second training sample set, model parameters of the part of the first initial model having the same structure, and second initial model parameters, a first round of model training, to obtain a second initial model, at least a part of model parameters of the second initial model being used to train the first machine learning model; and continue to perform, based on the second training sample set, model parameters of the part of the present first machine learning model having the same structure, and a part of the model parameters of the second machine learning model obtained from a previous round of model training, the model training, to obtain the second machine learning model.

In an embodiment, the whole of the first machine learning model has the same structure as the whole of the second machine learning model. The above second training module is specifically configured to perform, based on the second training sample set and all of the model parameters of the present first machine learning model, the M^{th} round of model training, to obtain the second machine learning model.

In an embodiment, the above second training module is configured to perform, if it is determined that the model index of the second machine learning model does not meet the second preset index, an (M+1)^{th} round of model training of the second machine learning model.

In an embodiment, the model index includes an accuracy of an output result, the first preset index includes a first preset accuracy, and the second preset index includes a second preset accuracy.

In an embodiment, the apparatus further includes:
a descent gradient module configured to determine, during each round of model training, a descent gradient using a batch gradient algorithm if it is determined, according to a preset loss function, that an output result of the machine learning model does not meet a preset convergence condition, and continue to perform the training, and stop, until it is determined that the output result of the machine learning model meets the preset convergence condition, the present round of model training.

In an embodiment, the above sample set acquisition module is specifically configured to: acquire medical images from the first hospital, and generate, based on the medical images from the first hospital, the first training sample set; and acquire medical images from the second hospital, and generate, based on the medical images from the second hospital, the second training sample set. The first hospital is different from the second hospital.

In an embodiment, the first machine learning model and the second machine learning model include at least one of a dose prediction model, an automatic sketching model, an efficacy evaluation model, a survival index evaluation model, a cancer screening model or a deformation registration model.

In an embodiment, the model parameters of the first machine learning model and the model parameters of the second machine learning model are transferred through a network.

Alternatively, the model parameters of the first machine learning model and the model parameters of the second machine learning model are transferred through a storage medium.

In an embodiment, the training of the first machine learning model and the training of the second machine learning model are performed on two independent networks, respectively.

In an embodiment, the training of the first machine learning model and the training of the second machine learning model are performed alternately.

In an embodiment, the first machine learning model and the second machine learning model are identical in structure and application.

In an embodiment, the first machine learning model and the second machine learning model are different in application.

In an embodiment, only model parameters are transferred during the training of the first machine learning model and the second machine learning model.

In an embodiment, the apparatus further includes:
a combination processing module configured to combine the first machine learning model and the second machine learning model to obtain a target machine learning model.

An apparatus for training machine learning models is provided. The apparatus includes:
a sample set acquisition module configured to acquire at least two training sample sets, training samples in the training sample set including medical images obtained by scanning a scan subject with a medical scanning device; and
a training module configured to perform, based on each of the at least two training sample sets, multiple rounds of model training, to obtain a machine learning model corresponding to a respective training sample set.

At least a part of each of at least two machine learning models has a same structure as at least a part of each of other machine learning models, and when one of the at least two machine learning models is trained, at least a part of model parameters of the part of another of the at least two machine learning models having the same structure is used.

In an embodiment, the above training module is configured to obtain model parameters for a present round for each of the machine learning models, and perform, based on the training sample set corresponding to the machine learning model and the model parameters for the present round, the model training, to obtain the machine learning model. The model parameters for the present round include initial model parameters, or the model parameters of the part of another machine learning model having the same structure.

In an embodiment, the training of each of the machine learning models is performed on an independent network.

In an embodiment, at least two of the machine learning models have the same configuration and the same application.

In an embodiment, each of the machine learning models has a different application.

A computer device is provided. The computer device includes a processor and a memory storing a computer program. The processor, when executing the computer program, implements the following steps:
acquiring a first training sample set and a second training sample set, training samples in the first training sample set and the second training sample set including medical images obtained by scanning a scan subject with a medical scanning device;
performing, based on the first training sample set, multiple rounds of model training, to obtain a first machine learning model; and
performing, based on the second training sample set, multiple rounds of model training, to obtain a second machine learning model.

At least a part of the first machine learning model has a same structure as at least a part of the second machine learning model, at least a part of model parameters of the second machine learning model is used when the first machine learning model is trained, and at least a part of model parameters of the first machine learning model is used when the second machine learning model is trained.

A computer-readable storage medium having a computer program stored thereon is provided. The computer program, when executed by a processor, implements the following steps:
acquiring a first training sample set and a second training sample set, training samples in the first training sample set and the second training sample set including medical images obtained by scanning a scan subject with a medical scanning device;
performing, based on the first training sample set, multiple rounds of model training, to obtain a first machine learning model; and
performing, based on the second training sample set, multiple rounds of model training, to obtain a second machine learning model.

At least a part of the first machine learning model has a same structure as at least a part of the second machine learning model, at least a part of model parameters of the second machine learning model is used when the first machine learning model is trained, and at least a part of model parameters of the first machine learning model is used when the second machine learning model is trained.

According to the above method and apparatus for training machine learning models, a computer device, and a storage medium, a first training sample set and a second training sample set are acquired, and then multiple rounds of model training are performed based on the first training sample set to obtain a first machine learning model, and multiple rounds of model training are performed based on the second training sample set to obtain a second machine learning model. Since the first machine learning model and the second machine learning model include parts having the same structure, the parts having the same structure may use the same model parameters. When the first machine learning model is trained, at least a part of the model parameters of the second machine learning model is used, and when the second machine learning model is trained, at least a part of the model parameters of the first machine learning model is used. In embodiments of the present disclosure, the first machine learning model is trained without using the second training sample set, and the second machine learning model is trained without using the first training sample set, which can ensure the data security of the training samples. While the first machine learning model is trained using the model parameters of the second machine learning model, and the second machine learning model is trained using the model parameters of the first machine learning model, which can improve the speed of the model training and the accuracy of the machine learning model.

### BRIEF DESCRIPTION OF THE DRAWINGS

FIG. 1 is a diagram illustrating an application environment of a method for training machine learning models in an embodiment.
FIG. 2 is a flow diagram illustrating a method for training machine learning models in an embodiment.
FIG. 3a is a schematic diagram illustrating a structure of a first machine learning model in an embodiment.
FIG. 3b is a schematic diagram illustrating a structure of a second machine learning model in an embodiment.
FIG. 4 is a flow diagram illustrating steps of performing multiple rounds of model training based on a first training sample set in an embodiment.
FIG. 5 is another flow diagram illustrating steps of performing multiple rounds of model training based on a first training sample set in an embodiment.
FIG. 6 is a flow diagram illustrating steps of performing multiple rounds of model training based on a second training sample set in an embodiment.
FIG. 7 is another flow diagram illustrating steps of performing multiple rounds of model training based on a second training sample set in an embodiment.
FIG. 8 is a flow diagram illustrating steps of alternately training a first machine learning model and a second machine learning model in an embodiment.
FIG. 9 is a flow diagram illustrating a method for training machine learning models in another embodiment.
FIG. 10 is a block diagram illustrating a configuration of an apparatus for training machine learning models in an embodiment.
FIG. 11 is a block diagram illustrating a configuration of an apparatus for training machine learning models in another embodiment.
FIG. 12 is a diagram illustrating an internal configuration of a computer device in an embodiment.

### DETAILED DESCRIPTION OF THE EMBODIMENTS

In order to make the objectives, technical solutions and advantages of the present disclosure more clearly understood, the present disclosure will be further described in detail with the accompanying drawings and embodiments. It should be understood that the specific embodiments described herein are only used to explain the present disclosure and not to limit the present disclosure.

First, before introducing the technical solutions according to the embodiments of the present disclosure in detail, the technical background or technological evolution context on which the embodiments of the present disclosure are based will be introduced. Generally, the training machine learning models requires a large number of training samples. However, since the medical images involve patient privacy and data security, and the medical images cannot be shared between hospitals, there are fewer training samples for the machine learning model, resulting in poor accuracy of the machine learning model.

The present disclosure provides a method for training machine learning models. The method includes: acquiring a first training sample set and a second training sample set; performing, based on the first training sample set, multiple rounds of model training, to obtain a first machine learning model; and performing, based on the second training sample set, multiple rounds of model training, to obtain a second machine learning model. At least a part of the first machine learning model has a same structure as at least a part of the second machine learning model. When the first machine learning model is trained, at least a part of model parameters of the second machine learning model is used, and when the second machine learning model is trained, at least a part of model parameters of the first machine learning model are used. In this way, the training of the first machine learning model does not use the second training sample set, and the training of the second machine learning model does not use the first training sample set, which can ensure the data security of the training samples. The training of the first machine learning model uses the model parameters of the second machine learning model, and the training of the second machine learning model uses the model parameters of the first machine learning model, which can improve the speed of the model training and the accuracy of the machine learning model. It can be seen that the problem in the conventional art of fewer training samples and poor accuracy of the machine learning model is solved.

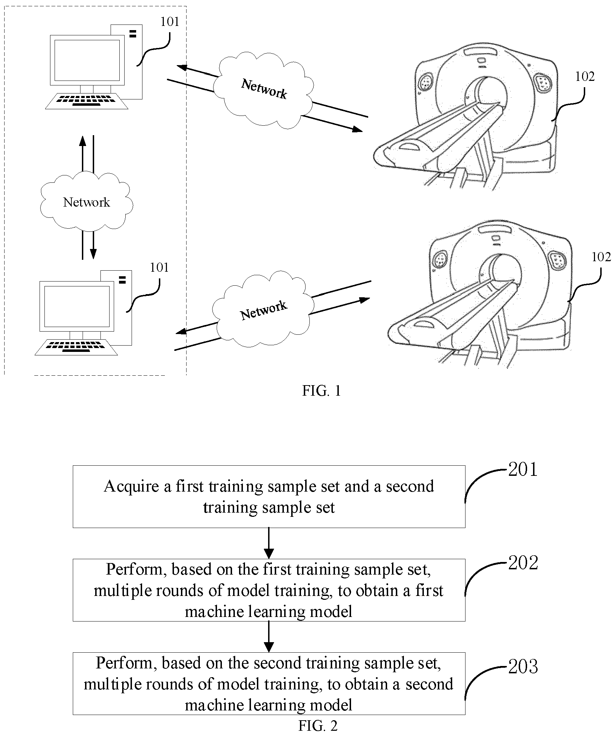

The method for training machine learning models provided by the present disclosure may be applied in an application environment as shown in FIG. 1. The application environment may include a model training system. The model training system includes a plurality of model training terminals 101. The plurality of model training terminals 101 may communicate with each other through a network. The model training terminal 101 may be a terminal connected to a medical scanning device 102. The terminal may be, but is not limited to, various personal computers, notebook computers, or tablet computers. The above medical scanning device 102 may be a single-mode device, or a multi-mode device. For example, the medical scanning device 102 may be, but is not limited to, a digital radiography (DR) equipment, a computed tomography (CT) equipment, a cone beam computed tomography (CBCT) equipment, a positron emission computed tomography (PET) equipment, a magnetic resonance imaging (MRI) equipment, an ultrasound equipment, a PET-CT equipment, a PET-MR equipment, a radiotherapy (RT) equipment, a CT-RT equipment or an MR-RT equipment. The model training terminal 101 may also be a picture archiving and communication systems (PACS) server. The above PACS server may be implemented as an independent server or a server cluster composed of a plurality of servers.

In an embodiment, as shown in FIG. 2, a method for training machine learning models is provided. Taking the method applied to the model training system shown in FIG. 1 as an example, the method includes the following steps.

In step 201, a first training sample set and a second training sample set are acquired.

Training samples in the first training sample set and the second training sample set include medical images obtained by scanning a scan subject with a medical scanning device. The medical image may include a two-dimensional image, or a three-dimensional image. For example, a CT image, a CBCT image, a PET image, an MR image, an ultrasound image, and the like. The training sample set satisfies data diversity, and label consistency, and the data structure is the same.

During the training of the machine learning model, the model training system may use medical images from the same hospital for model training, or use medical images from different hospitals for model training.

When the model training system uses the medical images from the same hospital for model training, the model training terminal generates the first training sample set and the second training sample set based on a plurality of medical images. The first training sample set and the second training sample set are used for different training tasks.

For example, the model training terminal acquires a hundred CT images from the same CT equipment. The hundred CT images are divided into two image sets to obtain the first training sample set and the second training sample set. The first training sample set is used for a training task for a dose prediction model, and the second training sample set is used for a training task for an automatic sketching model.

When the model training system uses the medical images from different hospitals for model training, a first model training terminal in the model training system acquires the medical images from a first hospital, and generates the first training sample set based on the medical images from the first hospital. A second model training terminal in the model training system acquires the medical images from a second hospital, and generates the second training sample set based on the medical images from the second hospital. The first hospital is different from the second hospital.

For example, the model training terminal A1 acquires CT images from a hospital B1 and generates the first training sample set. The model training terminal A2 acquires CT images from a hospital B2, and generates the second training sample set.

The embodiment of the present disclosure does not limit the method of acquiring the training sample set.

In step 202, multiple rounds of model training are performed based on the first training sample set to obtain a first machine learning model.

In step 203, multiple rounds of model training are performed based on the second training sample set to obtain a second machine learning model.

When the model training system uses the medical images from the same hospital for model training, the model training system may use the same model training terminal to train the first machine learning model and the second machine learning model, or use different model training terminals to train the first machine learning model and the second machine learning model.

When the model training system uses the medical images from different hospitals for model training, the model training system uses different model training terminals to train the first machine learning model and the second machine learning model.

In an embodiment, at least a part of the first machine learning model has a same structure as at least a part of the second machine learning model. At least a part of model parameters of the second machine learning model is used when the first machine learning model is trained, and at least a part of model parameters of the first machine learning model is used when the second machine learning model is trained.

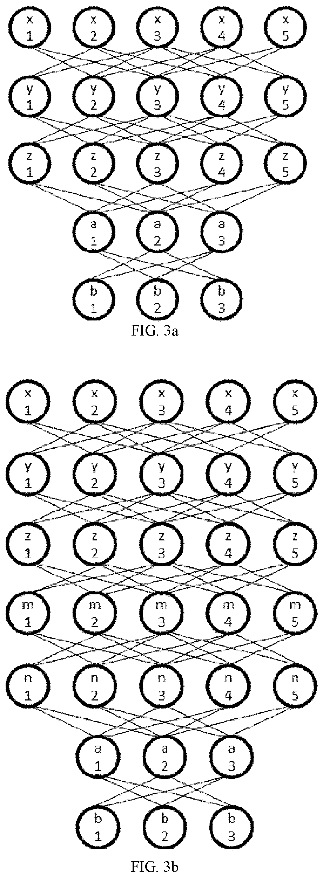

The first machine learning model as shown in FIG. 3a and the second machine learning model as shown in FIG. 3b have parts having the same structure. Therefore, the parts having the same structure may use the same model parameters. In this way, when the first machine learning model is trained, at least a part of the model parameters of the second machine learning model is used, and when the second machine learning model is trained, at least a part of the model parameters of the first machine learning model is used.

For example, the first model training terminal in the model training system performs a round of model training to obtain the model parameters of the first machine learning model, and then the model parameters of the first machine learning model are transferred to the second model training terminal in the model training system. The second model training terminal performs a round of model training of the second machine learning model using the model parameters of the first machine learning model. After the training is completed, the model parameters of the second machine learning model are transferred to the first model training terminal. The first model training terminal performs another round of model training of the first machine learning model using the model parameters of the second machine learning model. After the training is completed, the model parameters of the first machine learning model are transferred to the second model training terminal again. And so on, until the training of the first machine learning model and the second machine learning model is completed.

The model parameters of the first machine learning model and the model parameters of the second machine learning model are transferred through a network. Alternatively, the model parameters of the first machine learning model and the model parameters of the second machine learning model are transferred through a storage medium. For example, the first model training terminal and the second model training terminal send the model parameters to each other through the network, or the user copy the model parameters using the storage medium such as a mobile hard disk to realize the transfer of the model parameters, which is not limited thereto in the embodiments of the present disclosure.

Understandably, during the above model training, the first machine learning model is trained without using the second training sample set, and the second machine learning model is trained without using the first training sample set, which can ensure the data security of the training samples. The first machine learning model is trained using the model parameters of the second machine learning model, and the second machine learning model is trained using the model parameters of the first machine learning model, which can improve the speed of the model training and the accuracy of the machine learning model.

In an embodiment, the first machine learning model and the second machine learning model include at least one of a dose prediction model, an automatic sketching model, an efficacy evaluation model, a survival index evaluation model, a cancer screening model or a deformation registration model.

For example, the first machine learning model is the dose prediction model, and the second machine learning model is the automatic sketching model. Alternatively, the first machine learning model is the efficacy evaluation model, and the second machine learning model is the survival index evaluation model. The model types of the first machine learning model and the second machine learning model are not limited in the embodiments of the present disclosure.

For different hospitals, training samples are limited, so the accuracy of the application model obtained by training the model by using the limited samples in the conventional art is limited. In the training method of this embodiment, the model obtained by iterative training of the machine learning model by repeatedly using the limited samples has higher accuracy, which can achieve better technical effects when applied. For example, compared with the automatic sketching model obtained by training with fewer samples in the conventional art, the automatic sketching model obtained by using the training method of this embodiment has a better sketch effect on an area of interest. For another example, compared with the dose prediction model obtained by training with fewer samples in the conventional art, the dose prediction model obtained by using the training method of this embodiment may predict a dose more accurately.

In the above training method of the machine learning model, the first training sample set and the second training sample set are acquired, and then multiple rounds of model training are performed based on the first training sample set to obtain the first machine learning model, and multiple rounds of model training are performed based on the second training sample set to obtain the second machine learning model. Since the first machine learning model and the second machine learning model have parts having the same structure, the parts having the same structure may use the same model parameters. When the first machine learning model is trained, at least a part of the model parameters of the second machine learning model is used, and when the second machine learning model is trained, at least a part of the model parameters of the first machine learning model is used. In this embodiment of the present disclosure, the first machine learning model is trained without using the second training sample set, and the second machine learning model is trained without using the first training sample set, which can ensure the data security of the training samples. While the first machine learning model is trained using the model parameters of the second machine learning model, and the second machine learning model is trained using the model parameters of the first machine learning model, which can improve the speed of the model training and the accuracy of the machine learning model.

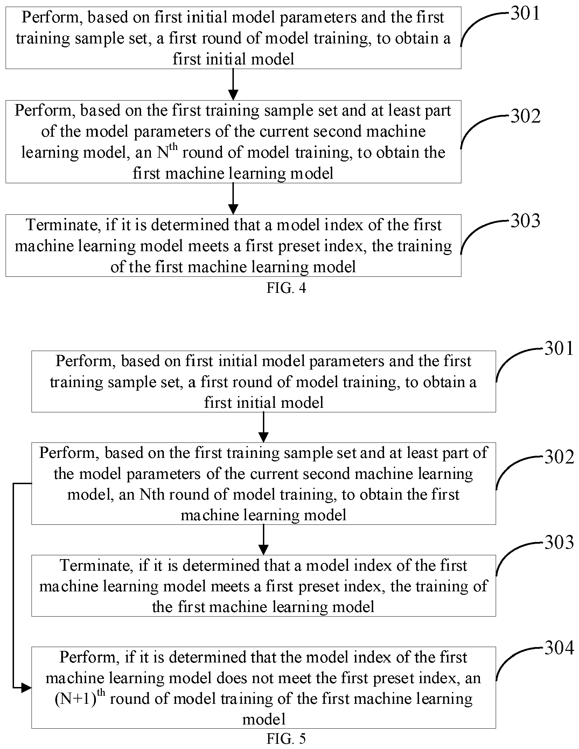

In an embodiment, as shown in FIG. 4, the above steps of performing multiple rounds of model training based on the first training sample set to obtain the first machine learning model may include the following steps.

In step 301, a first round of model training is performed based on first initial model parameters and the first training sample set to obtain a first initial model.

The first initial model parameters may be random model parameters, or user-assigned model parameters, which are not limited in this embodiment of the present disclosure.

After acquiring the first initial model parameters and the first training sample set, the model training terminal performs the first round of model training of the first machine learning model to obtain the first initial model. At least a part of the model parameters of the first initial model is used to train the second machine learning model.

In step 302, an N^{th} round of model training is performed based on the first training sample set and at least a part of the model parameters of the present second machine learning model to obtain the first machine learning model. N is a positive integer greater than 1.

The first machine learning model and the second machine learning model may be trained alternately. After obtaining the first initial model, the model training terminal performs a first round of model training of the second machine learning model using the model parameters of the first initial model.

Then, the model training terminal performs the N^{th} round of model training of the first machine learning model. In a case that a part of the first machine learning model has the same structure as a part of the second machine learning model, the performing, based on the first training sample set and at least a part of the model parameters of the present second machine learning model, the N^{th} round of model training, to obtain the first machine learning model may include: performing, based on the first training sample set, model parameters of the part of the present second machine learning model having the same structure, and a part of the model parameters of the first machine learning model obtained from a previous round of model training, the N^{th} round of model training, to obtain the first machine learning model.

Taking N equal to 2 as an example, the model training terminal performs a second round of model training of the first machine learning model based on the first training sample set, a part of the model parameters of the first initial model, and the model parameters of the part having the same structure of the second machine learning model obtained from the first round of model training, then the first machine learning model is obtained, and step 303 or step 304 is executed. Taking N equal to 3 as an example, the model training terminal performs a third round of model training of the first machine learning model based on the first training sample set, the first machine learning model trained in the second round, and the model parameters of the part having the same structure of the second machine learning model obtained from the second round of model training, then the first machine learning model is obtained, and step 303 or step 304 is executed.

In a case that the whole of the first machine learning model has the same structure as the whole of the second machine learning model, the performing, based on the first training sample set and at least a part of the model parameters of the present second machine learning model, the N^{th} round of model training, to obtain the first machine learning model may include: performing, based on the first training sample set and all of the model parameters of the present second machine learning model, the N^{th} round of model training, to obtain the first machine learning model.

In step 303, if it is determined that a model index of the first machine learning model meets a first preset index, the training of the first machine learning model is terminated.

After each round of model training, the model index of the first machine learning model is calculated, and it is determined whether the model index meets the first preset index. If the model index meets the first preset index, the training of the first machine learning model is terminated.

In an embodiment, the model index includes an accuracy of an output result, and the first preset index includes a first preset accuracy.

The model training terminal calculates the accuracy of the output result of the first machine learning model, and compares the accuracy of the output result of the first machine learning model with the first preset accuracy. If the accuracy of the output result of the first machine learning model is greater than the first preset accuracy, it is determined that the model index of the first machine learning model meets the first preset index. If the accuracy of the output result of the first machine learning model is less than or equal to the first preset accuracy, it is determined that the model index of the first machine learning model does not meet the first preset index.

The above process of calculating the accuracy of the output result of the first machine learning model may include inputting a preset number of test samples into the first machine learning model, to obtain the output results corresponding to respective test samples; counting the number of output results that are consistent with the labeling of the test samples, and calculating a ratio between this number and the preset number, to obtain the accuracy of the output results. The test samples may be selected from the first training sample set, or may be obtained using the same acquisition method as the first training sample set, which is not limited thereto in this embodiment of the present disclosure. Moreover, the preset number is not limited in this embodiment of the present disclosure.

In an embodiment, as shown in FIG. 5, the embodiment of the present disclosure may further include the following steps.

In step 304, if it is determined that the model index of the first machine learning model does not meet the first preset index, an (N+1)^{th} round of model training of the first machine learning model is performed.

After each round of model training, if the model index of the first machine learning model does not meet the first preset index, the next round of model training of the first machine learning model is performed.

Taking N equal to 2 as an example, if the model index of the first machine learning model does not meet the first preset index, at least a part of the model parameters obtained by training the second machine learning model in the second round are obtained. Then a third round of model training of the first machine learning model is performed based on the first training sample set, the first machine learning model trained in the second round, and at least a part of the model parameters obtained by training the second machine learning model in the second round, and step 303 or step 304 is performed.

Taking N equal to 3 as an example, if the model index of the first machine learning model does not meet the first preset index, at least a part of the model parameters obtained by training the second machine learning model in the third round are obtained. Then a fourth round of model training of the first machine learning model is performed based on the first training sample set, the first machine learning model trained in the third round, and at least a part of the model parameters obtained by training the second machine learning model in the third round, and step 303 or step 304 is performed.

In an embodiment, during each round of model training, if it is determined, according to a preset loss function, that the output result of the machine learning model does not meet a preset convergence condition, a descent gradient is determined using a batch gradient algorithm. The model training is continued until it is determined that the output result of the machine learning model meets the preset convergence condition, and the present round of model training is stopped.

For example, during the first round of model training, if it is determined that the output of the first machine learning model does not meet the preset convergence condition based on the preset loss function, the descent gradient is determined using the batch gradient descent method, and the model training is continued until it is determined that the output result of the first machine learning model meets the preset convergence condition, and the first round of model training is stopped. During the second round of model training, the preset loss function and the batch gradient descent method are also configured for model training. The preset loss function and the preset convergence condition are not limited in the embodiment of the present disclosure.

In the process of performing multiple rounds of model training based on the first training sample set to obtain the first machine learning model, the first round of model training is performed based on the first initial model parameters, and the first training sample set to obtain the first initial model, and the N^{th} round of model training is performed based on the first training sample set, and at least a part of the model parameters to obtain the first machine learning model. Then if it is determined that the model index of the first machine learning model meets the first preset index, the training of the first machine learning model is stopped, and if it is determined that the model index of the first machine learning model does not meet the first preset index, the (N+1)^{th} round of model training of the first machine learning model is performed. In the embodiment of the present disclosure, the first machine learning model that meets the first preset index may be trained by using at least a part of the model parameters of the second machine learning model without using the second training sample set. In this process, the data security of the training samples may be ensured, and the speed of the model training and the accuracy of the model may also be improved.

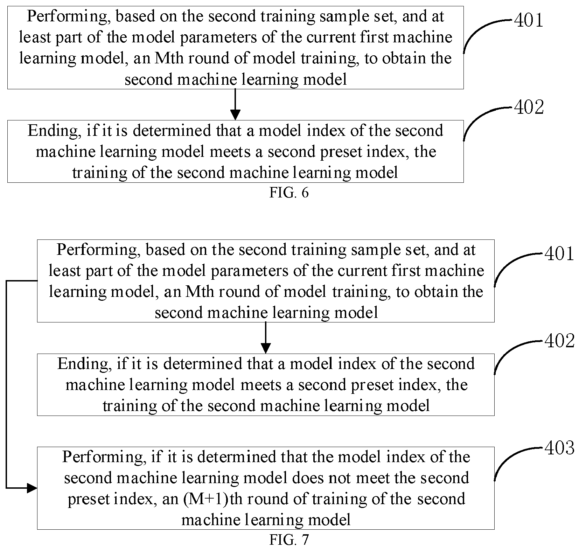

In an embodiment, as shown in FIG. 6, the above step of performing, based on the second training sample set, multiple rounds of model training, to obtain the second machine learning model may include the following steps.

In step 401, an M^{th} round of model training is performed based on the second training sample set, and at least a part of the model parameters of the present first machine learning model to obtain the second machine learning model. M being a positive integer greater than 0.

After obtaining the first initial model by training the first machine learning model, the model training terminal performs the M^{th} round of model training of the second machine learning model based on the second training sample set and at least a part of the model parameters of the first initial model.

In a case that a part of the first machine learning model has the same structure as a part of the second machine learning model, the performing, based on the second training sample set and the model parameters of the present first machine learning model, the M^{th} round of model training, to obtain the second machine learning model may include: performing, based on the second training sample set, model parameters of the part of the first initial model having the same structure, and second initial model parameters, a first round of model training, to obtain a second initial model, at least a part of model parameters of the second initial model being used to train the first machine learning model; and continuing to perform, based on the second training sample set, model parameters of the part of the present first machine learning model having the same structure, and a part of the model parameters of the second machine learning model obtained from a previous round of model training, the model training, to obtain the second machine learning model.

For example, after the first round of model training is completed, the model training terminal performs the second round of model training of the second machine learning model based on the second training sample set, the second machine learning model obtained from the first round of model training, and at least a part of the model parameters obtained by training the first machine learning model in the second round, and then the second machine learning model is obtained, and step 402 or step 403 is performed.

After the second round of model training is completed, if it is necessary to continue training, the model training terminal performs the third round of model training of the second machine learning model based on the second training sample set, the second machine learning model trained in the second round, and at least a part of the model parameters obtained by training the first machine learning model in the third round, and then the second machine learning model is obtained, and step 402 or step 403 is performed.

In a case that the whole of the first machine learning model has the same structure as the whole of the second machine learning model, the performing, based on the second training sample set and the model parameters of the present first machine learning model, the M^{th} round of model training, to obtain the second machine learning model may include performing, based on the second training sample set and all of the model parameters of the present first machine learning model, the M^{th} round of model training, to obtain the second machine learning model.

In step 402, if it is determined that a model index of the second machine learning model meets a second preset index, the training of the second machine learning model is terminated.

After each round of model training, the model index of the second machine learning model is calculated, and it is determined whether this model index meets the second preset index. If this model index meets the first preset index, the training of the second machine learning model is terminated.

In an embodiment, the model index includes an accuracy of an output result, and the second preset index includes a second preset accuracy.

In practical applications, the accuracy of the output result of the second machine learning model may be calculated, and the accuracy of the output result of the second machine learning model may be compared with the second preset accuracy. If the accuracy of the output result of the second machine learning model is greater than the second preset accuracy, it is determined that the model index of the second machine learning model meets the second preset index. If the accuracy of the output result of the second machine learning model is less than or equal to the second preset accuracy, it is determined that the model index of the second machine learning model does not meet the second preset index.

The above process of calculating the accuracy of the output result of the second machine learning model may include inputting a preset number of test samples into the second machine learning model, to obtain the output results corresponding to respective test samples; counting the number of output results that are consistent with the labeling of the test samples, and calculating a ratio between this number and the preset number, to obtain the accuracy of the output results. The test samples may be selected from the second training sample set, or may be obtained using the same acquisition method as the second training sample set, which is not limited thereto in this embodiment of the present disclosure. Moreover, the preset number is not limited in this embodiment of the present disclosure.

The above first preset index and the second preset index may be the same preset index, or may be different preset indexes, which are not limited in this embodiment of the present disclosure.

In an embodiment, as shown in FIG. 7, the embodiment of the present disclosure may further include the following steps.

In step 403, if it is determined that the model index of the second machine learning model does not meet the second preset index, an (M+1)^{th} round of model training of the second machine learning model is performed.

After each round of model training, if the model index of the second machine learning model does not meet the second preset index, the next round of model training of the second machine learning model is performed.

For example, after the second round of model training, if the model index of the second machine learning model does not meet the second preset index, the model parameters obtained by training the first machine learning model in the third round are obtained, and then the third round of model training of the second machine learning model is performed based on the second training sample set, the second machine learning model trained in the second round, and the model parameters obtained by training the first machine learning model in the third round.

After the third round of model training, if the model index of the second machine learning model does not meet the second preset index, the model parameters obtained by training the first machine learning model in the fourth round are obtained, and then the fourth round of model training of the second machine learning model is performed based on the second training sample set, the second machine learning model trained in the third round, and the model parameters obtained by training the first machine learning model in the fourth round. By analogy, until the model index of the second machine learning model meets the second preset index.

In an embodiment, during each round of model training, if it is determined, according to a preset loss function, that the output result of the machine learning model does not meet a preset convergence condition, a descent gradient is determined using a batch gradient algorithm. The model training is continued until it is determined that the output result of the machine learning model meets the preset convergence condition, and the present round of model training is stopped.

For example, during the first round of model training, if it is determined that the output of the second machine learning model does not meet the preset convergence condition based on the preset loss function, the descent gradient is determined using the batch gradient descent method, and the model training is continued until it is determined that the output result of the second machine learning model meets the preset convergence condition, and the first round of model training is stopped. During the second round of model training, the preset loss function and the batch gradient descent method are also configured for model training.

In the process of performing multiple rounds of model training based on the second training sample set to obtain the second machine learning model, the M^{th} round of model training is performed based on the second training sample set, and at least a part of the model parameters of the present first machine learning model to obtain the second machine learning model. Then if it is determined that the model index of the second machine learning model meets the second preset index, the training of the second machine learning model is stopped, and if it is determined that the model index of the second machine learning model does not meet the second preset index, the (M+1)^{th} round of model training of the second machine learning model is performed. In the embodiment of the present disclosure, the second machine learning model that meets the second preset index may be trained by using the model parameters of the first machine learning model without using the first training sample set. In this process, the data security of the training samples may be ensured, and the speed of the model training and the accuracy of the model may also be improved.

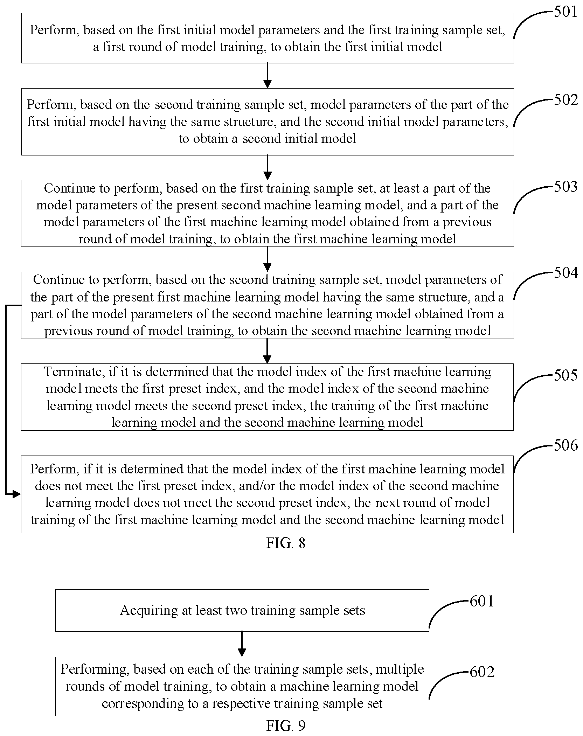

In an embodiment, the training of the first machine learning model and the training of the second machine learning model are performed alternately. In a case that a part of the first machine learning model has the same structure as a part of the second machine learning model, as shown in FIG. 8, the following steps may be included.

In step 501, a first round of model training is performed based on the first initial model parameters and the first training sample set to obtain the first initial model.

In step 502, a first round of model training is performed based on the second training sample set, model parameters of the part of the first initial model having the same structure, and the second initial model parameters to obtain a second initial model.

The same structure includes the same model hierarchy, the same connection relationship, etc.

In step 503, the model training is continued to be performed based on the first training sample set, at least a part of the model parameters of the present second machine learning model, and a part of the model parameters of the first machine learning model obtained from a previous round of model training, to obtain the first machine learning model.

In step 504, the model training is continued to be performed based on the second training sample set, model parameters of the part of the present first machine learning model having the same structure, and a part of the model parameters of the second machine learning model obtained from a previous round of model training, to obtain the second machine learning model.

In step 505, if it is determined that the model index of the first machine learning model meets the first preset index, and the model index of the second machine learning model meets the second preset index, then the training of the first machine learning model and the second machine learning model is terminated.

In step 506, if it is determined that the model index of the first machine learning model does not meet the first preset index, and/or the model index of the second machine learning model does not meet the second preset index, then the next round of model training of the first machine learning model and the second machine learning model is performed.

In an embodiment, the first machine learning model is the same as the second machine learning model in structure. In this case, in the step 503, the model training is continued to be performed based on the first training sample set and all of the model parameters of the present second machine learning model to obtain the first machine learning model. In the step 504, the model training is continued to be performed based on the second training sample set and all of the model parameters of the present first machine learning model to obtain the second machine learning model.

In an embodiment, the training of the first machine learning model and the training of the second machine learning model are performed on two independent networks, respectively.

For example, the first model training terminal and the second model training terminal in the model training system are placed in two independent networks, and the first model training terminal and the second training terminal do not communicate with each other through a network. In this case, the model parameters of the first machine learning model and the model parameters of the second machine learning model may be transferred through a storage medium.

In an embodiment, only model parameters are transferred during the training of the first machine learning model and the second machine learning model.

In an embodiment, the first machine learning model and the second machine learning model are identical in structure and application.

For example, the first machine learning model is a dose prediction model trained using the first training sample set, and the second machine learning model is a dose prediction model trained using the second training sample set. The first machine learning model and the second machine learning model have the same configuration, and both are applied to dose prediction.

In an embodiment, the first machine learning model and the second machine learning model are different in application.

For example, the first machine learning model is a dose prediction model, the second machine learning model is an automatic sketching model, and the structures of the first machine learning model and the second machine learning model may be partially or completely the same.

In an embodiment, the embodiment of the present disclosure may further include combining the first machine learning model and the second machine learning model to obtain a target machine learning model.

The model training terminal combines the first machine learning model and the second machine learning model to obtain the combined target machine learning model. For example, by combining the dose prediction model with the automatic sketching model, a target machine learning model that performs dose prediction first followed by automatic sketching may be obtained, making the function of the model more powerful.

During the above model training, the first machine learning model and the second machine learning model are trained alternately. The first machine learning model is trained without using the second training sample set, and the second machine learning model is trained without using the first training sample set, which can ensure the data security of training samples. The first machine learning model is trained using the model parameters of the second machine learning model, and the second machine learning model is trained using the model parameters of the first machine learning model, which can improve the speed of the model training and the accuracy of the machine learning model.

In an embodiment, as shown in FIG. 9, a method for training machine learning models is provided. Taking the method applied to the model training system shown in FIG. 1 as an example, the method includes the following steps.

In step 601, at least two training sample sets are acquired.

Training samples in the training sample set include medical images obtained by scanning a scan subject with a medical scanning device. For example, the medical images include CT images, CBCT images, PET images, MR images, ultrasound images, and so on. The model training system acquires at least two training sample sets, and the acquisition method may refer to step 201.

In step 602, multiple rounds of model training are performed based on each of the training sample sets, to obtain a machine learning model corresponding to a respective training sample set.

At least a part of each of at least two machine learning models has a same structure as at least a part of each of other machine learning models, and when one of the machine learning models are trained, at least a part of model parameters with same configuration of another machine learning model are used.

For each of the machine learning models, model parameters for a present round are obtained, and the model training is performed based on the training sample set corresponding to the machine learning model and the model parameters for the present round, to obtain the machine learning model. The model parameters for the present round include initial model parameters, or the model parameters of the part of another machine learning model having the same structure.

For example, three training sample sets are acquired to train three machine learning models. At least a part of the first machine learning model has a same structure as at least a part of the second machine learning model, at least a part of the second machine learning model has a same structure as at least a part of the third machine learning model, and at least a part of the first machine learning model has a same structure as at least a part of the third machine learning model. During the training, for the first machine learning model, initial model parameters of the first round are obtained, and then a first round of model training is performed based on the first training sample set and the initial model parameters. For the second machine learning model, at least a part of the model parameters of the first machine learning model are obtained, and a first round of model training is performed based on the second training sample set and at least a part of the model parameters of the first machine learning model. For the third machine learning model, at least a part of the model parameters of the second machine learning model are obtained, and a first round of model training is performed based on the third training sample set and at least a part of the model parameters of the second machine learning model.

After the first round, for the first machine learning model, at least a part of the model parameters of the third machine learning model are obtained, and a second round of model training is performed based on the first training sample set and at least a part of the model parameters of the third machine learning model. For the second machine learning model, at least a part of the model parameters of the first machine learning model are obtained, and a second round of model training is performed based on the second training sample set and at least a part of the model parameters of the first machine learning model. For the third machine learning model, at least a part of the model parameters of the second machine learning model are obtained, and a second round of model training is performed based on the third training sample set and at least a part of the model parameters of the second machine learning model. By analogy, the training is performed in sequence.

During the training, the model parameters are transferred between at least two machine learning models in a preset order. For example, at least a part of the model parameters of the first machine learning model are transferred to the second machine learning model, at least a part of the model parameters of the second machine learning model are transferred to the third machine learning model, and at least a part of the model parameters of the third machine learning model are transferred to the first machine learning model. In other embodiments, the transfer of the model parameters may also be in other transfer forms, and the preset order is not limited in the embodiment of the present disclosure.

In an embodiment, the training of each of the machine learning models is performed on an independent network.

For example, the first machine learning model, the second machine learning model, and the third machine learning model are trained on three independent networks, and there is no communication between the three networks.

In an embodiment, at least two of the machine learning models are identical in structure and application.

For example, the first machine learning model, the second machine learning model, and the third machine learning model are all dose prediction models, and the structures of the first machine learning model, the second machine learning model, and the third machine learning model are the same.

In an embodiment, each of the machine learning models has a different application.

For example, the first machine learning model is a dose prediction model, the second machine learning model is an automatic sketching model, and the third machine learning model is an efficacy evaluation model. The structures of each two of the machine learning models may be partially or completely the same.

In the above method for training machine learning models, at least two training sample sets are acquired, and then multiple rounds of model training are performed based on each of the training sample sets to obtain a machine learning model corresponding to a respective training sample set. Since structures of each two of the machine learning models are at least partially the same, when one of the machine learning models is trained, at least a part of the model parameters of the part having the same structure of another machine learning model are used, the data security of training samples can be ensured, and the speed of model training and the accuracy of machine learning models can be improved.

It should be understood that although the individual steps in the flow diagrams shown in FIGS. 2 to 9 above are shown sequentially as indicated by arrows, the steps are not necessarily performed sequentially in the order indicated by the arrows. Unless explicitly stated herein, the execution of these steps is not strictly limited in order and these steps can be performed in any other order. Moreover, at least some of the steps shown in FIGS. 2 to 9 may include a plurality of steps or a plurality of stages that are not necessarily performed at the same time, but may be performed at different times. The order in which these steps or stages are performed is not necessarily sequential, and these steps may be performed alternately or alternately with other steps or at least some of the steps or stages in other steps.

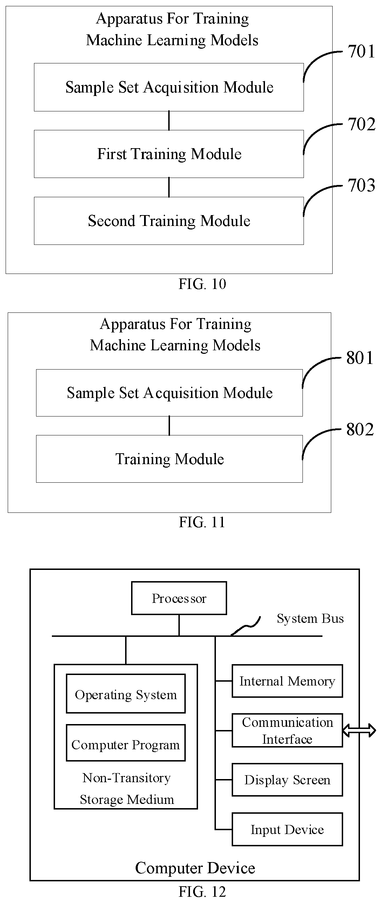

In an embodiment, as shown in FIG. 10, an apparatus for training machine learning models is provided, including:
a sample set acquisition module 701 configured to acquire a first training sample set and a second training sample set, training samples in the first training sample set and the second training sample set including medical images obtained by scanning a scan subject with a medical scanning device;
a first training module 702 configured to perform, based on the first training sample set, multiple rounds of model training, to obtain a first machine learning model; and
a second training module 703 configured to perform, based on the second training sample set, multiple rounds of model training, to obtain a second machine learning model.

At least a part of the first machine learning model has a same structure as at least a part of the second machine learning model, at least a part of model parameters of the second machine learning model is used when the first machine learning model is trained, and at least a part of model parameters of the first machine learning model is used when the second machine learning model is trained.

In an embodiment, the above first training module 702 is specifically configured to perform, based on first initial model parameters and the first training sample set, a first round of model training, to obtain a first initial model, at least a part of model parameters of the first initial model being used to train the second machine learning model; perform, based on the first training sample set and at least a part of the model parameters of the present second machine learning model, an N^{th} round of model training, to obtain the first machine learning model, N being a positive integer greater than 1; and terminate, if it is determined that a model index of the first machine learning model meets a first preset index, the training of the first machine learning model.

In an embodiment, a part of the first machine learning model has the same structure as a part of the second machine learning model. The above first training module 702 is specifically configured to perform, based on the first training sample set, model parameters of the part of the present second machine learning model having the same structure, and a part of the model parameters of the first machine learning model obtained from a previous round of model training, the N^{th} round of model training, to obtain the first machine learning model.

In an embodiment, the whole of the first machine learning model has the same structure as the whole of the second machine learning model. The above first training module 702 is specifically configured to perform, based on the first training sample set and all of the model parameters of the present second machine learning model, the N^{th} round of model training, to obtain the first machine learning model.

In an embodiment, the above first training module 702 is further configured to perform, if it is determined that the model index of the first machine learning model does not meet the first preset index, an (N+1)^{th} round of model training of the first machine learning model.

In an embodiment, the above second training module 703 is specifically configured to perform, based on the second training sample set, and at least a part of the model parameters of the present first machine learning model, an M^{th} round of model training, to obtain the second machine learning model, M being a positive integer greater than 0; and terminate, if it is determined that a model index of the second machine learning model meets a second preset index, the training of the second machine learning model.

In an embodiment, a part of the first machine learning model has the same structure as a part of the second machine learning model. The above second training module 703 is specifically configured to perform, based on the second training sample set, model parameters of the part of the first initial model having the same structure, and second initial model parameters, a first round of model training, to obtain a second initial model, at least a part of model parameters of the second initial model being used to train the first machine learning model; and continue to perform, based on the second training sample set, model parameters of the part of the present first machine learning model having the same structure, and a part of the model parameters of the second machine learning model obtained from a previous round of model training, the model training, to obtain the second machine learning model.

In an embodiment, the whole of the first machine learning model has the same structure as the whole of the second machine learning model. The above second training module 703 is specifically configured to perform, based on the second training sample set and all of the model parameters of the present first machine learning model, the M^{th} round of model training, to obtain the second machine learning model.

In an embodiment, the above second training module 703 is configured to perform, if it is determined that the model index of the second machine learning model does not meet the second preset index, an (M+1)^{th} round of model training of the second machine learning model.

In an embodiment, the model index includes an accuracy of an output result, the first preset index includes a first preset accuracy, and the second preset index includes a second preset accuracy.

In an embodiment, the apparatus further includes:
a descent gradient module configured to determine, during each round of model training, a descent gradient using a batch gradient algorithm if it is determined, according to a preset loss function, that an output result of the machine learning model does not meet a preset convergence condition, and continue to perform the training, and stop, until it is determined that the output result of the machine learning model meets the preset convergence condition, the present round of model training.

In an embodiment, the above sample set acquisition module 701 is specifically configured to: acquire medical images from the first hospital, and generate, based on the medical images from the first hospital, the first training sample set; and acquire medical images from the second hospital, and generate, based on the medical images from the second hospital, the second training sample set. The first hospital is different from the second hospital.

In an embodiment, the first machine learning model and the second machine learning model include at least one of a dose prediction model, an automatic sketching model, an efficacy evaluation model, a survival index evaluation model, a cancer screening model or a deformation registration model.

In an embodiment, the model parameters of the first machine learning model and the model parameters of the second machine learning model are transferred through a network.

Alternatively, the model parameters of the first machine learning model and the model parameters of the second machine learning model are transferred through a storage medium.

In an embodiment, the training of the first machine learning model and the training of the second machine learning model are performed on two independent networks, respectively.

In an embodiment, the training of the first machine learning model and the training of the second machine learning model are performed alternately.

In an embodiment, the first machine learning model and the second machine learning model are identical in structure and application.

In an embodiment, the first machine learning model and the second machine learning model are different in application.

In an embodiment, only model parameters are transferred during the training of the first machine learning model and the second machine learning model.

In an embodiment, as shown in FIG. 11, an apparatus for training machine learning models is provided. The apparatus includes:
a sample set acquisition module 801 configured to acquire at least two training sample sets, training samples in the training sample set including medical images obtained by scanning a scan subject with a medical scanning device; and
a training module 802 is configured to perform, based on each of the at least two training sample sets, multiple rounds of model training, to obtain a machine learning model corresponding to a respective training sample set.

At least a part of each of at least two machine learning models has a same structure as at least a part of each of other machine learning models, and when one of the at least two machine learning models is trained, at least a part of model parameters of the part of another of the at least two machine learning models having the same structure is used.

In an embodiment, the above training module 802 is configured to obtain model parameters for a present round for each of the machine learning models, and perform, based on the training sample set corresponding to the machine learning model and the model parameters for the present round, the model training, to obtain the machine learning model. The model parameters for the present round include initial model parameters, or the model parameters of the part of another machine learning model having the same structure.

In an embodiment, the training of each of the machine learning models is performed on an independent network.

In an embodiment, at least two of the machine learning models have the same configuration and the same application.

In an embodiment, each of the machine learning models has a different application.

The specific features in the apparatus for training machine learning models may be understood with reference to the features of the method for training machine learning models above and will not be repeated here. The individual modules in the above apparatus for training machine learning models can be implemented in whole or in part by software, hardware and combinations thereof. Each of the above modules may be embedded in hardware form or independent of a processor in a computer device, or may be stored in software form on a memory in the computer device so that the processor can be called to perform the operations corresponding to each of the above modules.

In an embodiment, a computer device is provided, which may be a terminal. A diagram illustrating an internal configuration of the computer device is shown in FIG. 12. The computer device includes a processor, a memory, a communication interface, a display screen, and an input device connected through a system bus. The processor of the computer device is configured to provide computing and control capabilities. The memory of the computer device includes a non-transitory storage medium and an internal memory. The non-transitory storage medium stores operating systems and computer programs. The internal memory provides an environment for the operation of operating systems and computer programs in non-transitory storage medium. The communication interface of the computer device is configured to communicate with external terminals in wired or wireless mode, which can be realized by WIFI, mobile cellular network, near field communication (NFC) or other technologies. The computer programs are executed by the processor in order to implement a method for training machine learning models. The display screen of the computer device may be an LCD or e-ink display, and the input device of the computer device may be a touch layer covered by the display screen, or a key, trackball or trackpad set on the housing of the computer device, or an external keyboard, trackpad or mouse, etc.

It should be understood by those skilled in the art that the configuration illustrated in FIG. 12, which is only a block diagram of part of the configuration related to the solution of the present disclosure, and does not constitute a limitation on the computer device to which the solution of the present disclosure is applied. The specific computer device may include more or less components than those shown in the figure, or may combine some components, or may have a different arrangement of components.

In an embodiment, a computer device is provided, including a processor and a memory storing a computer program. The processor, when executing the computer program, implements the following steps.

A first training sample set and a second training sample set are acquired. Training samples in the first training sample set and the second training sample set include medical images obtained by scanning a scan subject with a medical scanning device.

Multiple rounds of model training are performed based on the first training sample set to obtain a first machine learning model.

Multiple rounds of model training are performed based on the second training sample set to obtain a second machine learning model.

At least a part of the first machine learning model has a same structure as at least a part of the second machine learning model, at least a part of model parameters of the second machine learning model is used when the first machine learning model is trained, and at least a part of model parameters of the first machine learning model is used when the second machine learning model is trained.

In an embodiment, the processor, when executing the computer program, further implements the following steps.

A first round of model training is performed based on first initial model parameters and the first training sample set to obtain a first initial model. At least a part of model parameters of the first initial model is configured to train the second machine learning model.

An N^{th} round of model training is performed based on the first training sample set and at least a part of the model parameters of the present second machine learning model to obtain the first machine learning model. N is a positive integer greater than 1.

If it is determined that a model index of the first machine learning model meets a first preset index, the training of the first machine learning model is terminated.

In an embodiment, a part of the first machine learning model has the same structure as a part of the second machine learning model. The processor, when executing the computer program, further implements the following steps.

The N^{th} round of model training is performed based on the first training sample set, model parameters of the part of the present second machine learning model having the same structure, and a part of the model parameters of the first machine learning model obtained from a previous round of model training to obtain the first machine learning model.

In an embodiment, the whole of the first machine learning model has the same structure as the whole of the second machine learning model. The processor, when executing the computer program, further implements the following steps.

The N^{th} round of model training is performed based on the first training sample set and all of the model parameters of the present second machine learning model to obtain the first machine learning model.

In an embodiment, the processor, when executing the computer program, further implements the following steps.

If it is determined that the model index of the first machine learning model does not meet the first preset index, an (N+1)^{th} round of model training of the first machine learning model is performed.

In an embodiment, the processor, when executing the computer program, further implements the following steps.

An M^{th} round of model training is performed based on the second training sample set, and at least a part of the model parameters of the present first machine learning model to obtain the second machine learning model. M is a positive integer greater than 0.

If it is determined that a model index of the second machine learning model meets a second preset index, the training of the second machine learning model is terminated.

In an embodiment, a part of the first machine learning model has the same structure as a part of the second machine learning model. The processor, when executing the computer program, further implements the following steps.

A first round of model training is performed based on the second training sample set, model parameters of the part of the first initial model having the same structure, and second initial model parameters to obtain a second initial model. At least a part of model parameters of the second initial model are configured to train the first machine learning model.

The model training is continued to be performed based on the second training sample set, model parameters of the part of the present first machine learning model having the same structure, and a part of the model parameters of the second machine learning model obtained from a previous round of model training, to obtain the second machine learning model.

In an embodiment, the whole of the first machine learning model has the same structure as the whole of the second machine learning model. The processor, when executing the computer program, further implements the following steps.

The M^{th} round of model training is performed based on the second training sample set and all of the model parameters of the present first machine learning model to obtain the second machine learning model.

In an embodiment, the processor, when executing the computer program, further implements the following steps.

If it is determined that the model index of the second machine learning model does not meet the second preset index, an (M+1)^{th} round of model training of the second machine learning model is performed.

In an embodiment, the model index includes an accuracy of an output result, the first preset index includes a first preset accuracy, and the second preset index includes a second preset accuracy.

In an embodiment, the processor, when executing the computer program, further implements the following steps.

During each round of model training, if it is determined, according to a preset loss function, that an output result of the machine learning model does not meet a preset convergence condition, a descent gradient is determined using a batch gradient algorithm. The model training is continued until it is determined that the output result of the machine learning model meets the preset convergence condition, and the present round of model training is stopped.

In an embodiment, the processor, when executing the computer program, further implements the following steps.

Medical images from the first hospital are acquired, and the first training sample set is generated based on the medical images from the first hospital.

Medical images from the second hospital are acquired, and the second training sample set is generated based on the medical images from the second hospital.

The first hospital is different from the second hospital.

In an embodiment, the first machine learning model and the second machine learning model include at least one of a dose prediction model, an automatic sketching model, an efficacy evaluation model, a survival index evaluation model, a cancer screening model or a deformation registration model.

In an embodiment, the model parameters of the first machine learning model and the model parameters of the second machine learning model are transferred through a network.

Alternatively, the model parameters of the first machine learning model and the model parameters of the second machine learning model are transferred through a storage medium.

In an embodiment, the training of the first machine learning model and the training of the second machine learning model are performed on two independent networks, respectively.

In an embodiment, the training of the first machine learning model and the training of the second machine learning model are performed alternately.

In an embodiment, the first machine learning model and the second machine learning model are identical in structure and application.

In an embodiment, the first machine learning model and the second machine learning model are different in application.

In an embodiment, only model parameters are transferred during the training of the first machine learning model and the second machine learning model.

In an embodiment, the processor, when executing the computer program, further implements the following steps.

The first machine learning model is combined with the second machine learning model to obtain a target machine learning model.

In an embodiment, a computer-readable storage medium is provided, having a computer program stored thereon. The computer program, when executed by a processor, implements the following steps.

A first training sample set and a second training sample set are acquired. Training samples in the first training sample set and the second training sample set include medical images obtained by scanning a scan subject with a medical scanning device.

Multiple rounds of model training are performed based on the first training sample set to obtain a first machine learning model.

Multiple rounds of model training are performed based on the second training sample set to obtain a second machine learning model.

At least a part of the first machine learning model has a same structure as at least a part of the second machine learning model, at least a part of model parameters of the second machine learning model is used when the first machine learning model is trained, and at least a part of model parameters of the first machine learning model is used when the second machine learning model is trained.

In an embodiment, the computer program, when executed by the processor, further implements the following steps.

A first round of model training is performed based on first initial model parameters and the first training sample set to obtain a first initial model. At least a part of model parameters of the first initial model is configured to train the second machine learning model.

An N^{th} round of model training is performed based on the first training sample set and at least a part of the model parameters of the present second machine learning model to obtain the first machine learning model. N is a positive integer greater than 1.

If it is determined that a model index of the first machine learning model meets a first preset index, the training of the first machine learning model is terminated.

In an embodiment, a part of the first machine learning model has the same structure as a part of the second machine learning model. The computer program, when executed by the processor, further implements the following steps.

The N^{th} round of model training is performed based on the first training sample set, model parameters of the part of the present second machine learning model having the same structure, and a part of the model parameters of the first machine learning model obtained from a previous round of model training to obtain the first machine learning model.

In an embodiment, the whole of the first machine learning model has the same structure as the whole of the second machine learning model. The computer program, when executed by the processor, further implements the following steps.

The N^{th} round of model training is performed based on the first training sample set and all of the model parameters of the present second machine learning model to obtain the first machine learning model.

In an embodiment, the computer program, when executed by the processor, further implements the following steps.

If it is determined that the model index of the first machine learning model does not meet the first preset index, an (N+1)^{th} round of model training of the first machine learning model is performed.

In an embodiment, the computer program, when executed by the processor, further implements the following steps.

An M^{th} round of model training is performed based on the second training sample set, and at least a part of the model parameters of the present first machine learning model to obtain the second machine learning model. M is a positive integer greater than 0.

If it is determined that a model index of the second machine learning model meets a second preset index, the training of the second machine learning model is terminated.

In an embodiment, a part of the first machine learning model has the same structure as a part of the second machine learning model. The computer program, when executed by the processor, further implements the following steps.

A first round of model training is performed based on the second training sample set, model parameters of the part of the first initial model having the same structure, and second initial model parameters to obtain a second initial model. At least a part of model parameters of the second initial model are configured to train the first machine learning model.

The model training is continued to be performed based on the second training sample set, model parameters of the part of the present first machine learning model having the same structure, and a part of the model parameters of the second machine learning model obtained from a previous round of model training, to obtain the second machine learning model.

In an embodiment, the whole of the first machine learning model has the same structure as the whole of the second machine learning model. The computer program, when executed by the processor, further implements the following steps.

The M^{th} round of model training is performed based on the second training sample set and all of the model parameters of the present first machine learning model to obtain the second machine learning model.

In an embodiment, the computer program, when executed by the processor, further implements the following steps.

If it is determined that the model index of the second machine learning model does not meet the second preset index, an (M+1)^{th} round of model training of the second machine learning model is performed.

In an embodiment, the model index includes an accuracy of an output result, the first preset index includes a first preset accuracy, and the second preset index includes a second preset accuracy.

In an embodiment, the computer program, when executed by the processor, further implements the following steps.

During each round of model training, if it is determined, according to a preset loss function, that an output result of the machine learning model does not meet a preset convergence condition based on a preset loss function, a descent gradient is determined using a batch gradient algorithm, and the model training is continued until it is determined that the output result of the machine learning model meets the preset convergence condition, the present round of model training is stopped.

In an embodiment, the computer program, when executed by the processor, further implements the following steps.

Medical images from the first hospital are acquired, and the first training sample set is generated based on the medical images from the first hospital.

Medical images from the second hospital are acquired, and the second training sample set is generated based on the medical images from the second hospital.

The first hospital is different from the second hospital.

In an embodiment, the first machine learning model and the second machine learning model include at least one of a dose prediction model, an automatic sketching model, an efficacy evaluation model, a survival index evaluation model, a cancer screening model or a deformation registration model.

In an embodiment, the model parameters of the first machine learning model and the model parameters of the second machine learning model are transferred through a network.

Or the model parameters of the first machine learning model and the model parameters of the second machine learning model are transferred through a storage medium.

In an embodiment, the training of the first machine learning model and the training of the second machine learning model are performed on two independent networks, respectively.

In an embodiment, the training of the first machine learning model and the training of the second machine learning model are performed alternately.

In an embodiment, the first machine learning model and the second machine learning model are identical in structure and application.

In an embodiment, the first machine learning model and the second machine learning model are different in application.

In an embodiment, only model parameters are transferred during the training of the first machine learning model and the second machine learning model.

In an embodiment, the computer program, when executed by the processor, further implements the following steps.

The first machine learning model is combined with the second machine learning model to obtain a target machine learning model.

A person of ordinary skill in the art may understand that implementation of all or part of the processes in the methods of the above embodiments may be completed by instructing the relevant hardware through a computer program. The computer program may be stored in a non-transitory computer-readable storage medium. When the computer program is executed, it may include the processes of the embodiments of the above methods. Any reference to memory, database or other medium used of the embodiments provided in the present disclosure may include at least one of a non-transitory and a transitory memory. The non-transitory memory may include a read-only memory (ROM), a magnetic tape, a floppy disk, a flash memory, or an optical memory. The transitory memory may include a random-access memory (RAM) or an external cache memory, etc. As an illustration rather than a limitation, the random-access memory may be in various forms, such as a static random-access memory (SRAM) or a dynamic random-access memory (DRAM), etc.

The technical features in the above embodiments may be combined arbitrarily. For concise description, not all possible combinations of the technical features in the above embodiments are described. However, provided that they do not conflict with each other, all combinations of the technical features are to be considered to be within the scope described in this specification.

The above-mentioned embodiments only describe several implementations of the present disclosure, and their description is specific and detailed, but should not be understood as a limitation on the patent scope of the present disclosure. It should be noted that, for a person of ordinary skill in the art may further make variations and improvements without departing from the conception of the present disclosure, and these all fall within the protection scope of the present disclosure. Therefore, the protection scope of the present disclosure should be subject to the appended claims.

## Claims

1. A method for training machine learning models, the method comprising:
acquiring a first training sample set and a second training sample set, training samples in the first training sample set and the second training sample set comprising medical images obtained by scanning a scan subject with a medical scanning device;
performing, based on the first training sample set, multiple rounds of model training, to obtain a first machine learning model; and
performing, based on the second training sample set, multiple rounds of model training, to obtain a second machine learning model,
wherein at least a part of the first machine learning model has a same structure as at least a part of the second machine learning model, at least a part of model parameters of the second machine learning model is used when the first machine learning model is trained, and at least a part of model parameters of the first machine learning model is used when the second machine learning model is trained.

2. The method of claim 1, wherein the performing, based on the first training sample set, multiple rounds of model training, to obtain the first machine learning model comprises:
performing, based on first initial model parameters and the first training sample set, a first round of model training, to obtain a first initial model, at least a part of model parameters of the first initial model being used to train the second machine learning model;
performing, based on the first training sample set and at least a part of the model parameters of the present second machine learning model, an N^{th} round of model training, to obtain the first machine learning model, N being a positive integer greater than 1; and
terminating, if it is determined that a model index of the first machine learning model meets a first preset index, the training of the first machine learning model.

3. The method of claim 2, wherein a part of the first machine learning model has the same structure as a part of the second machine learning model, and the performing, based on the first training sample set and at least a part of the model parameters of the present second machine learning model, the N^{th} round of model training, to obtain the first machine learning model comprises:
performing, based on the first training sample set, model parameters of the part of the present second machine learning model having the same structure, and a part of the model parameters of the first machine learning model obtained from a previous round of model training, the N^{th} round of model training, to obtain the first machine learning model.

4. The method of claim 2, wherein the whole of the first machine learning model has the same structure as the whole of the second machine learning model, and the performing, based on the first training sample set and at least a part of the model parameters of the present second machine learning model, the N^{th} round of model training, to obtain the first machine learning model comprises:
performing, based on the first training sample set and all of the model parameters of the present second machine learning model, the N^{th} round of model training, to obtain the first machine learning model.

5. The method of claim 2, further comprising:
performing, if it is determined that the model index of the first machine learning model does not meet the first preset index, an (N+1)^{th} round of model training of the first machine learning model.

6. The method of claim 2, wherein the performing, based on the second training sample set, multiple rounds of model training, to obtain the second machine learning model comprises:
performing, based on the second training sample set, and at least a part of the model parameters of the present first machine learning model, an M^{th} round of model training, to obtain the second machine learning model, M being a positive integer greater than 0; and
terminating, if it is determined that a model index of the second machine learning model meets a second preset index, the training of the second machine learning model.

7. The method of claim 6, wherein a part of the first machine learning model has the same structure as a part of the second machine learning model, and the performing, based on the second training sample set and at least a part of the model parameters of the present first machine learning model, the M^{th} round of model training, to obtain the second machine learning model comprises:
performing, based on the second training sample set, model parameters of the part of the first initial model having the same structure, and second initial model parameters, a first round of model training, to obtain a second initial model, at least a part of model parameters of the second initial model being used to train the first machine learning model; and
continuing to perform, based on the second training sample set, model parameters of the part of the present first machine learning model having the same structure, and a part of the model parameters of the second machine learning model obtained from a previous round of model training, the model training, to obtain the second machine learning model.

8. The method of claim 6, wherein the whole of the first machine learning model has the same structure as the whole of the second machine learning model, and the performing, based on the second training sample set and at least a part of the model parameters of the present first machine learning model, the M^{th} round of model training, to obtain the second machine learning model comprises:
performing, based on the second training sample set and all of the model parameters of the present first machine learning model, the M^{th} round of model training, to obtain the second machine learning model.

9. The method of claim 6, further comprising:
performing, if it is determined that the model index of the second machine learning model does not meet the second preset index, an (M+1)^{th} round of model training of the second machine learning model.

10. The method of claim 6, wherein the model index comprises an accuracy of an output result, the first preset index comprises a first preset accuracy, and the second preset index comprises a second preset accuracy.

11. The method of claim 6, wherein the method further comprises:
determining, during each round of model training, a descent gradient using a batch gradient algorithm if it is determined, according to a preset loss function, that an output result of the machine learning model does not meet a preset convergence condition, and
continuing to perform the model training, and stopping, until it is determined that the output result of the machine learning model meets the preset convergence condition, the present round of model training.

12. The method of claim 1, wherein the acquiring the first training sample set and the second training sample set comprises:
acquiring medical images from a first hospital, and generating, based on the medical images from the first hospital, the first training sample set; and
acquiring medical images from a second hospital, and generating, based on the medical images from the second hospital, the second training sample set,
wherein the first hospital is different from the second hospital.

13. The method of claim 1, wherein the first machine learning model and the second machine learning model comprise at least one of a dose prediction model, an automatic sketching model, an efficacy evaluation model, a survival index evaluation model, a cancer screening model or a deformation registration model.

14. The method of claim 1, wherein the model parameters of the first machine learning model and the model parameters of the second machine learning model are transferred through a network, or
wherein the model parameters of the first machine learning model and the model parameters of the second machine learning model are transferred through a storage medium.

15. The method of claim 1, wherein the training of the first machine learning model and the training of the second machine learning model are performed on two independent networks, respectively.

16. The method of claim 1, wherein the training of the first machine learning model and the training of the second machine learning model are performed alternately.

17. The method of claim 1, wherein the first machine learning model and the second machine learning model are identical in structure and application.

18. The method of claim 1, wherein the first machine learning model and the second machine learning model are different in application.

19. The method of claim 1, wherein only model parameters are transferred during the training of the first machine learning model and the second machine learning model.

20. The method of claim 1, further comprising:
combining the first machine learning model and the second machine learning model, to obtain a target machine learning model.

21. A method for training machine learning models, the method comprising:
acquiring at least two training sample sets, training samples in the training sample set comprising medical images obtained by scanning a scan subject with a medical scanning device; and
performing, based on each of the at least two training sample sets, multiple rounds of model training, to obtain a machine learning model corresponding to a respective training sample set;
wherein at least a part of each of at least two machine learning models has a same structure as at least a part of each of other machine learning models, and when one of the at least two machine learning models is trained, at least a part of model parameters of the part of another of the at least two machine learning models having the same structure is used.

22. The method of claim 21, wherein the performing, based on each of the at least two training sample sets, multiple rounds of model training, to obtain a machine learning model corresponding to a respective training sample set comprises:
obtaining model parameters for a present round for each of the at least two machine learning models, and
performing, based on the training sample set corresponding to the machine learning model and the model parameters for the present round, the model training, to obtain the machine learning model,
wherein the model parameters for the present round comprise initial model parameters, or the model parameters of the part of another machine learning model having the same structure.

23. The method of claim 21, wherein the training of each of the machine learning models is performed on an independent network.

24. The method of claim 21, wherein the at least two machine learning models are identical in structure and application.

25. The method of claim 21, wherein each of the machine learning models are different in application.

26. An apparatus for training machine learning models, the apparatus comprising:
a sample set acquisition module configured to acquire a first training sample set and a second training sample set, training samples in the first training sample set and the second training sample set comprising medical images obtained by scanning a scan subject with a medical scanning device;
a first training module configured to perform, based on the first training sample set, multiple rounds of model training, to obtain a first machine learning model; and
a second training module configured to perform, based on the second training sample set, multiple rounds of model training, to obtain a second machine learning model,
wherein at least a part of the first machine learning model has a same structure as at least a part of the second machine learning model, at least a part of model parameters of the second machine learning model is used when the first machine learning model is trained, and at least a part of model parameters of the first machine learning model is used when the second machine learning model is trained.

27. An apparatus for training machine learning models, the apparatus comprising:
a sample set acquisition module configured to acquire at least two training sample sets, training samples in the training sample set comprising medical images obtained by scanning a scan subject with a medical scanning device; and
a training module configured to perform, based on each of the at least two training sample sets, multiple rounds of model training, to obtain a machine learning model corresponding to a respective training sample set;
wherein at least a part of each of at least two machine learning models has a same structure as at least a part of each of other machine learning models, and when one of the at least two machine learning models is trained, at least a part of model parameters of the part of another of the at least two machine learning models having the same structure is used.

28. A computer device comprising a processor, and a memory storing a computer program, wherein the processor, when executing the computer program, implements steps of the method of any one of claims 1 to 25.

29. A computer-readable storage medium having a computer program stored thereon, wherein the computer program, when executed by a processor, implements steps of the method of any one of claims 1 to 25.
